# EUROPÄISCHE PATENTSCHRIFT

(11) **EP 1 437 051 B1**
(45) Veröffentlichungstag und Bekanntmachung des Hinweises auf die Patenterteilung: **29.12.2010**
(21) Anmeldenummer: 04100040.7
(22) Anmeldetag: 08.01.2004
(51) Int. Cl.: A23L 1/302, A23L 1/304, A61K 31/375, A61K 31/355, A61K 33/30, A61K 33/04, A23L 1/30

(54) **Ocuvite Lutein**
Ocuvite Lutein
Lutéine ocuvite

(30) Priorität: 09.01.2003 DE 20300305 U
(43) Veröffentlichungstag der Anmeldung: 14.07.2004
(73) Patentinhaber: Dr. GERHARD MANN chem.-pharm. Fabrik GmbH, D-13581 Berlin (DE)
(72) Erfinder: Bellmann, Günther Dr., 13593 Berlin (DE); Claus-Herz, Gudrun Dr., 14167 Berlin (DE)
(74) Vertreter: Maiwald Patentanwalts GmbH

(56) Entgegenhaltungen:
- EP-A- 1 214 893
- WO-A-01/19383
- WO-A-03/063848
- GB-A- 2 301 775
- US-A- 5 976 568
- US-A1- 2002 182 266

## Beschreibung

Es ist allgemein bekannt, dass das Auge ein Hochleistungsorgan ist, das durch den ständigen Sehprozess hohen Belastungen ausgesetzt ist. Durch Licht- und Sauerstoffeinwirkung entstehen sogenannte "freie Radikale", die zu krankhaften Veränderungen in der Netzhaut führen können.

Es ist allgemein bekannt, dass viele äußere Einflüsse, wie z. B. eine unausgewogene Ernährung, Rauchen und Alkoholgenuss, Umweltverschmutzung, Stress oder UV-Strahlung, zu einer Freisetzung von Radikalen führen können. Aber auch die normalen Vorgänge im Stoffwechsel setzen kurzfristig hochaktive Substanzen frei, die den Körper belasten und schädigen können. Zum Abbau dieser "freien Radikale" besitzt der Körper ein eigenes "antioxidatives" Schutzsystem, an dem die Vitamine C und E, die vitaminähnlichen Carotinoide Lutein und Zeaxanthin sowie die Spurenelemente Selen und Zink entscheidend beteiligt sind. Einen optimalen Schutz bietet das System nur dann, wenn alle seine aktiven Bestandteile in ausreichender Menge vorhanden sind.

Das Auge ist durch den Sehprozess und die ständige Lichteinwirkung in erhöhtem Maß oxidativem Stress ausgesetzt. Es hat daher einen erhöhten Bedarf nach einer ausreichenden Versorgung mit den antioxidativen Substanzen Vitamin C, Vitamin E, Zink und den vitaminähnlichen Carotinoiden Lutein und Zeaxanthin.

In jüngeren Lebensjahren wird der Bedarf an Mikronährstoffen durch eine ausgewogene Ernährung weitgehend gesichert. Dies ist allerdings mit steigendem Lebensalter durch die Ernährung schwerer zu gewährleisten. Zu den Organen, welche eine besonders hohe Konzentrationen an den Carotinoiden Lutein und Zeaxanthin benötigen, zählt die Makula des Auges. Unter dem medizinischen Begriff "Makula lutea" ist die Netzhautmitte, also der Punkt des schärfsten Sehens zu verstehen. Hier befinden sich die empfindlichsten Sehzellen des Auges.

Bei einer länger anhaltenden Mangelversorgung der Makula mit Mikronährstoffen steigt das Risiko der Entwicklung einer "altersbedingten Makuladegeneration" (AMD). Die AMD hat ein Schwinden der Sehkraft zur Folge, im schlimmsten Fall bis hin zur Erblindung. Bei den über 65-Jährigen ist die altersbedingte Makuladegeneration die Hauptursache für Sehkraftverlust und Blindheit.

Die im Stand der Technik bekannten Vitaminpräparate enthalten verschiedene, häufig überdosierte, Zusammensetzungen von Vitaminen und Spurenelementen, die jedoch nicht auf eine Versorgung der Makula des Auges ausg erichtet sind. So enthalten ein Großteil dieser Zusammensetzungen hohe Anteile an antioxidativ wirkenden Vitaminen und Spurenelementen, jedoch nicht Lutein und Zeaxanthin. Ein Nachteil dieser Präparate ist ihre mangelnde Berücksichtigung der Makula, die eine zentrale Funktion beim Sehprozess hat.

Ein weiterer Nachteil handelsüblicher antioxidativer Präparate besteht darin, dass die Altersabhängigkeit des Vitamin - und Mineralstoffbedarfs zu wenig berücksichtigt wird. Besonders im Alter können sich Mangelers cheinungen durch Unterversorgung aufgrund von Mangel- oder Fehlernährung oder Krankheit rasch bemerkbar machen, insbesondere im Hinblick auf Erkrankungen wie z. B. AMD, denen durch darauf abgestimmte Präparate zu wenig Rechnung getragen wird.

Erhältliche antioxidative Präparate sind vielfach Vitamin C-Monopräparate, die in hohen Dosierungen verabreicht werden und vielfach keine weiteren Spurenelemente oder Vitamine enthalten. Diese Präparate haben den Nachteil, dass sie für eine Dauertherapie beziehungsweise für eine zeitlich unbegrenzte Prophylaxe nicht geeignet sind, da diese Dosen für eine langandauernde Einnahme zu hoch liegen oder in der hohen Dosierung zu Problemen mit der Verträglichkeit führen können. Zudem führt eine hochdosierte Aufnahme zwar zu einer kurzfristig erhöhten Aufnahme, insgesamt wird jedoch ein unverhältnismäßig hoher Anteil des Wirkstoffs aufgrund des großen Überschusses nicht aufgenommen.

GB 2 301 775 offenbart die Verwendung von Lutein und Zeaxanthin entweder separat oder in Kombination zur Behandlung Altersbedingter Makuladegeneration (AMD).

WO 01/19383 offenbart Kügelchen, umfassend Xanthophylle und Carotine und/oder Retinoide, Nahrungsergänzungsmittel, umfassend diese Kügelchen und Verfahren zu deren Verwendung.

US 2002/0182266 offenbart Nahrungsergänzungsmittel, deren wesentliche Inhaltsstoffe Vitamin C, Vitamin E, Betacarotin, Zink und Kupfer sind.

US 5,976,568 offenbart ein modulares System von Nahrungsergänzungsmitteln. Insbesondere betrifft US 5,976,568 ein modulares Gesamtsystem von Multivitamin- und Mineralergänzungsmitteln, bestehend aus sieben unterschiedlichen Modulen zum Verbessern der öffentlichen Gesundheit durch Sicherstellen einer angemessenen Aufnahme von Mikronährstoffen, die nötig sind zum Verhüten von Erkrankungen und Schützen gegen Nährstoffverluste und Nährstoffdefizite.

EP 1 214 893 offenbart Zusammensetzungen zum Verbessern der Gesundheit, umfassend mindestens (a) 800 µg Vitamin A, 1000 mg Vitamin C, 400 IU Vitamin E, 200 µg Vitamin K (b) 10 mg Betacarotin, 6 mg Lutein, 5 mg Lycopin, 100 µg Zeaxanthin, (c) 7,5 mg Vitamin B1, 7,5 mg Vitamin B2, 15 mg Niacin, 15 mg Pantothensäure, 7,5 mg Vitamin B6, 200 µg Folsäure, 6,75 µg Vitamin B12 (d) 200 µg Selen, 10 mg Zink, 120 µg Chrom, 2 mg Kupfer, 4 mg Mangan, 100 µg Jod, 100 µg Molybdän, (e) 200 µg Biotin, 450 mg Betain, 100 mg Oligo Proanthocyanidine (OPC), 150 mg Polyphenolkomplex, 600-5000 mg gemischte Omega-3 und 6, 4-6 g Oligosaccharide, insbesondere Oligofructose und/oder Beta-Glucan, 4-6 g Inulin, und gegebenenfalls zusätzliche Substanzen zum Zwecke der Stabilisierung und Formulierung der vorstehend genannten Verbindungen.

Es besteht daher Bedarf an einem Mittel, das einer Mangelversorgung der Makula mit Mikronährstoffen entgegenwirkt. Darüber hinaus besteht Bedarf an einem Mittel, das als solches keine gesundheitsschädliche Nebenwirkung hervorruft und sowohl unbedenklich zur Präventionen von Mangelerscheinungen dienen kann, wie auch bei bereits bestehender altersbedingter Makuladegeneration verwendbar ist.

Es ist daher die Aufgabe der vorliegenden Erfindung, ein Mittel zur Verfügung zu stellen, das die vorgenannten Nachteile des Standes der Technik überwindet. Insbesondere ist es die Aufgabe der vorliegenden Erfindung, ein Mittel zur Verfügung zu stellen, das eine umfassende Versorgung der Makula bietet.

Diese Aufgabe wird durch ein Mikronährstoffkombinationsprodukt gelöst, wobei das Mikronährstoffkombinationsprodukt,
die Wirkstoffe umfasst:
a. 0,6 - 1,2 g Lutein;
b. 0,05 - 0,1 g Zeaxanthin;
c. 5,6 - 11,2 g Vitamin C;
d. 0,8 - 2 g Vitamin E;
e. 2,0 - 3,0 mg Selen;
f. 0,5 - 1 g Zink.

Es hat sich überraschend gezeigt, dass die Makula durch die optimierte Kombination der vitaminähnlichen Carotinoide Lutein und Zeaxanthin deutlich gestärkt wurde. Es hat sich weiterhin überraschend gezeigt, dass die erfindungsgemäß optimiert verwendeten vitaminähnlichen Carotinoide durch ihre Fähigkeit, vorhandene Radikale abzufangen, einen verbesserten vorbeugenden Schutz der Makula bewirken.

Ohne auf eine bestimmte Theorie festgelegt zu sein wird angenommen, dass sich die vitaminähnlichen Carotinoide Lutein und Zeaxanthin in hohen Konzentrationen in der Makula selektiv anreichern und so diesen Bereich durch das Filtern des Lichts und das Abfangen der schädlichen "freien Radikale" schützen. Die gezielte Zufuhr dieser essenziellen Mikronährstoffen kann folglich dazu beitragen, das Auge vor der Entstehung einer altersbedingten Makuladegeneration zu schützen oder deren Fortschreiten zu verlangsamen.

Das erfindungsgemäße Mikronährstoffkombinationsprodukt ist kein Arzneimittel. Es kann zur unterstützenden nutritiven Ergänzung oder zur begleitenden Verabreichung beispielsweise bei der Behandlung einer altersbedingten Makuladegeneration verwendet werden.

Es kann auch eine Ausführungsform des Mikronährstoffkombinationsproduktes erfindungsgemäß geeignet sein, die frei von Beta-Carotin und/oder Kupferoxid ist. Eine orale Einnahme von Beta-Carotin kann insbesondere bei Rauchern in hohen Dosierungen zu Schädigungen in Verbindung mit Arzneimitteln führen. Das erfindungsgemäße Mikronährstoffkombinationsprodukt kann frei von Beta-Carotin sein. In vorteilhafter Weise wird durch eine Ausführungsform des Mikronährstoffkombinationsproduktes, die frei von Beta-Carotin ist, die Verträglichkeit des Mikronährstoffkombinationsproduktes insbesondere für Raucher positiv beeinflusst. Das erfindungsgemäße Mikronährstoffkombinationsprodukt kann auch frei von Kupferoxid sein.

Es hat sich überraschend gezeigt, dass die erfindungsgemäße Verwendung des Zeaxanthins kombiniert mit Zink die Versorgung der Konsumenten gegenüber der Verwendung von Monopräparaten positiv beeinflusst. In seiner Funktion als Nahrungsergänzungsmittel weist das erfindungsgemäße Mikronährstoffkombinationsprodukt Zeaxanthin auf, das in ausgewogener Kombination mit Lutein, dem Spurenelement Zink und weiteren antioxidativ wirkenden Vitaminen durch eine ausgeglichene Versorgung der Makula zur Vorbeugung einer Schädigung durch Radikale zur Wirkung des erfindungsgemäßen Mittels beiträgt, wodurch die Verbesserung der Sehfähigkeit im Alter positiv beeinflusst wird und die Entstehung einer altersbedingten Makuladegeneration erschwert wird. Durch die erfindungsgemäße Verwendung der vitaminähnlichen Carotinoide mit antioxidierenden Eigenschaften gemeinsam mit Zink weist das Mikronährstoffkombinationsprodukt eine verbesserte nutritive bzw. diätetische Wirkung auf.

Die vorteilhaften Eigenschaften ergeben sich aus der optimierten Auswahl der einzelnen Komponenten, die speziell zur Bekämpfung oxidativer Schädigungen der Makula durch Radikale ausgewählt wurden und ein durch Emährungsdefizite oder Risikofaktoren, wie z. B. Rauchen und Alkoholgenuss, Umweltverschmutzung, Stress oder UV-Strahlung, verursachtes Defizit an Antioxidantien ausgleichen. Besonders vorteilhaft ist, dass durch den optimierten Gehalt der Spurenelemente Zink und Selen eine deutliche Verbesserung der antioxidativen Eigenschaften der vitaminähnlichen Carotinoide Lutein und Zeaxanthin und der Vitamine C und E eintritt.

Ein weiterer Vorteil des erfindungsgemäßen Mikronährstoffkombinationsproduktes besteht in der synergistischen Wirkung im Hinblick auf eine umfassende ganzheitliche Versorgung der Makula. Die Makula wird durch die optimierte Kombination der Vitamine und Spurenelemente, insbesondere der vitaminähnlichen Carotinoide Lutein und Zeaxanthin und Zink gestärkt.

Vorteilhaft ist darüber hinaus, dass die Dosierungen des erfindungsgemäßen Mittels keine schädigenden Nebenwirkungen verursachen und zur Prophylaxe wie auch zum Ausgleich einer bestehenden Mangelversorgung der Makula dienen können. Dies ist zur nutritiven wie auch zur diätetischen Behandlung bei altersbedingter Makuladegeneration vorteilhaft.

Das erfindungsgemäße Mikronährstoffkombinationsprodukt kann durch eine Kombination der Spurenelemente Zink und Selen die antioxidativen Eigenschaften der Carotinoide Lutein und Zeaxanthin, und der Vitamine C und E unterstützen. Ein positiver Effekt zeigt sich insbesondere durch eine unterstützende nutritive oder diätetische Verabreichung bei Mangelzuständen und der nutritiven Vorbeugung der Entstehung einer Mangelsituation, die zu einer altersbedingten Makuladegeneration führen kann.

Das Mikronährstoffkombinationsprodukt kann fest-, flüssig und/oder gelförmig vorliegen, vorzugsweise liegt das Mikronährstoffkombinationsprodukt in Präparatformen, ausgewählt aus der Gruppe umfassend Tabletten, Kapseln, Pulver, Granulat, Lösungen und/oder Brausetabletten mit gleicher oder unterschiedlicher Zusammensetzung vor.

Das Mikronährstoffkombinationsprodukt kann Zusätze aufweisen, die die Bioverfügbarkeit, Löslichkeit und/oder Lösegeschwindigkeit verbessern. Ferner kann das Mikronährstoffkombinationsprodukt Sprengmittel, Stoffe, die die Haltbarkeit verbessern, geschmacksmaskierende Stoffe, geschmacksverbessernde Stoffe, Füllstoffe, Überzugsmittel und/oder Formentrennmittel aufweisen.

Geeignet sind übliche, dem Fachmann bekannten Zusätze, bevorzugte Füllstoffe sind beispielsweise Cellulose und/oder Lactose, bevorzugte Formentrennmittel sind Metallseifen, beispielsweise Magnesiumstearat. Zur Maskierung oder Verbesserung des Geschmacks können Stoffe ausgewählt aus der Gruppe umfassend natürliche, naturidentische oder synthetische Aromastoffe, Essenzen, ätherische Öle, natürliche oder synthetische Glutamate, Inosate, Guanylate und/oder Glycin oder deren Mischungen eingesetzt werden. Bevorzugt zur Verbesserung des Geschmacks sind Fruchtaromen. Weitere bevorzugte Zusätze sind Farbstoffe, Stabilisatoren und/oder Süßungsmittel.

In einer bevorzugten Ausführungsform umfasst das Mikronährstoffkombinationsprodukt, bezogen auf 100 g des Mikronährstoffkombinationsproduktes, die Wirkstoffe:
a. 0,6 g Lutein;
b. 0,05 g Zeaxanthin;
c. 5,6 g Vitamin C;
d. 0,8 g Vitamin E;
e. 2,0 mg Selen;
f. 0,5 g Zink.

In einer weiteren bevorzugten Ausführungsform umfasst das Mikronährstoffkombinationsprodukt, bezogen auf eine Tagesdosis, die Wirkstoffe:
a. 6,0 mg Lutein;
b. 0,5 mg Zeaxanthin;
c. 60,0 mg Vitamin C;
d. 8,8 mg Vitamin E;
e. 20 µg Selen;
f. 5 mg Zink.

Das Mikronährstoffpräparat kann auch Zusätze, Begleitstoffe und/oder Rohstoffe enthalten, die schwerer sind als die eigentlichen Wirkstoffe, so dass die angegebenen Mengen bezogen auf eine Tagesdosis bzw. Einzeldosis niedriger oder auch höher sein können.

In einer bevorzugten Ausführungsform umfasst das Mikronährstoffkombinationsprodukt, bezogen auf eine Einzeldosis, die Wirkstoffe:
a. 3,0 mg Lutein;
b. 0,25 mg Zeaxanthin;
c. 30,0 mg Vitamin C;
d. 4,4 mg Vitamin E;
e. 10 µg Selen;
f. 2,5 mg Zink.

In einer bevorzugten Ausführungsform weist das Mikronährstoffkombinationsprodukt, bezogen auf eine Einzeldosis, 0,02 g Proteine, 0,06 g Fett und/oder 0,19 g Kohlenhydrate auf. Weiterhin weist das Mikronährstoffkombinationsprodukt, bezogen auf eine Einzeldosis, bevorzugt 0,02 Broteinheiten auf.

Unter einer Einzeldosis wird im Sinne dieser Anmeldung eine Verabreichungseinheit des Mikronährstoffkombinationsproduktes verstanden, eine Einzeldosis des Mikronährstoffkombinationsproduktes entspricht beispielsweise einer Tablette, einer Kapsel oder einer einzelnen Verabreichungseinheit einer anderen Präparatform, wie Pulver oder Granulat. Unter einer Tagesdosis wird im Sinne dieser Anmeldung die Menge des Mikronährstoffkombinationsproduktes verstanden, die pro Tag verabreicht wird.

Die Tagesdosis und/oder Einzeldosis ist vorzugsweise auf mehrere gleiche oder unterschiedliche Präparatformen verteilt, wobei die Präparatformen gleiche oder unterschiedliche Wirkstoffe und/oder Wirkstoffgewichtsgehalte aufweisen können.

Das Mikronährstoffkombinationsprodukt kann zur Herstellung eines Mittels zur diätetischen Verhütung und Behandlung von Augenkrankheiten, bevorzugt bei altersbedingter Makuladegeneration, verwendet werden. Das erfindungsgemäße Mikronährstoffkombinationsprodukt ist weiterhin geeignet als Mittel zur Nahrungsergänzung.

Der Begriff "diätetische Behandlung" ist in der EU -Richtlinie 1999/21/EG, die seit 01.01.2002 als 10. Verordnung zur Änderung der Diätverordnung in deutsches Recht umgesetzt worden ist, näher erläutert.

Durch die vorteilhaften Wirkstoffe des Mikronährstoffkombinationsprodukts kann dieses auch im Rahmen einer diätetischen Emährungsberatung als Lebensmittel, insbesondere Nahrungsergänzungsmittel eingesetzt werden. Bevorzugt kann das Mikronährstoffkombinationsprodukt als nutritive Ergänzung zur begleitenden Verabreichung bei altersbedingter Makuladegeneration eingesetzt werden. Die Behandlung kann nutritiv unterstützt werden. Es hat sich gezeigt, dass insbesondere ein drohender Nährstoffmangel bei älteren Menschen ausgeglichen werden kann.

Beispiele für Mikronährstoffkombinationsprodukte sind nachstehend angegeben:

Es versteht sich, dass die Präparatformen, ausgewählt aus der Gruppe umfassend Tabletten, Kapseln, Pulver, Granulat, Lösungen und/oder Brausetabletten, die üblichen zur Formulierung der jeweiligen Präparatformen eingesetzten Hilfsstoffe aufweisen, so dass in den Beispielen lediglich die enthaltenen Wirkstoffe aufgeführt sind.

### Beispiel 1

Mikronährstoffkombinationsprodukt umfassend 1 Tablette mit folgenden Wirkstoffen:
- 3,0 mg Lutein;
- 0,25 mg Zeaxanthin;
- 30,0 mg Vitamin C;
- 4,4 mg Vitamin E;
- 10 µg Selen;
- 2,5 mg Zink.

### Beispiel 2

Mikronährstoffkombinationsprodukt, Tagesdosis umfassend 2 Tabletten mit jeweils folgenden Wirkstoffen:
- 3,0 mg Lutein;
- 0,25 mg Zeaxanthin;
- 30,0 mg Vitamin C;
- 4,4 mg Vitamin E;
- 10 µg Selen;
- 2,5 mg Zink.

## Patentansprüche

1. Mikronährstoffkombinationsprodukt, **dadurch gekennzeichnet, dass** das Mikronährstoffkombinationsprodukt, bezogen auf 100 g des Mikronährstoffkombinationsproduktes, die Wirkstoffe umfasst:
a. 0,6 - 1,2 g Lutein;
b. 0,05 - 0,1 g Zeaxanthin;
c. 5,6 - 11,2 g Vitamin C;
d. 0,8 - 2 g Vitamin E;
e. 2,0 - 3,0 mg Selen;
f. 0,5 - 1 g Zink.

2. Mikronährstoffkombinationsprodukt nach Anspruch 1, **dadurch gekennzeichnet, dass** das Mikronährstoffkombinationsprodukt fest-, flüssig und/oder gelförmig vorliegt, und wobei vorzugsweise das Mikronährstoffkombinationsprodukt in Präparatformen, ausgewählt aus der Gruppe umfassend Tabletten, Kapseln, Pulver, Granulat, Lösungen und/oder Brausetabletten vorliegt.

3. Mikronährstoffkombinationsprodukt nach Anspruch 1 oder 2, **dadurch gekennzeichnet, dass** das Mikronährstoffkombinationsprodukt Zusätze, die die Bioverfügbarkeit, Löslichkeit und/oder Lösegeschwindigkeit verbessern, aufweist.

4. Mikronährstoffkombinationsprodukt nach einem der vorherigen Ansprüche, **dadurch gekennzeichnet, dass** das Mikronährstoffkombinationsprodukt Sprengmittel, Stoffe, die die Haltbarkeit verbessern, geschmacksmaskierende Stoffe, geschmacksverbessernde Stoffe, Füllstoffe, Überzugsmittel und/oder Formentrennmittel aufweist.

5. Mikronährstoffkombinationsprodukt nach einem der vorherigen Ansprüche, **dadurch gekennzeichnet, dass** das Mikronährstoffkombinationsprodukt, bezogen auf 100 g des Mikronährstoffkombinationsproduktes, die Wirkstoffe umfasst:
a. 0,6 g Lutein;
b. 0,05 g Zeaxanthin;
c. 5,6 g Vitamin C;
d. 0,8 g Vitamin E;
e. 2,0 mg Selen;
f. 0,5 g Zink.

6. Mikronährstoffkombinationsprodukt nach einem der vorherigen Ansprüche, **dadurch gekennzeichnet, dass** das Mikronährstoffkombinationsprodukt, bezogen auf eine Einzeldosis, die Wirkstoffe umfasst:
a. 3,0 mg Lutein;
b. 0,25 mg Zeaxanthin
c. 30,0 mg Vitamin C;
d. 4,4 mg Vitamin E;
e. 10 µg Selen;
f. 2,5 mg Zink.

7. Verwendung eines Mikronährstoffkombinationsproduktes nach einem der vorherigen Ansprüche zur Herstellung eines Mittels zur diätetischen Verhütung und Behandlung von Augenkrankheiten, bevorzugt bei altersbedingter Makuladegeneration.

8. Verwendung eines Mikronährstoffkombinationsproduktes nach einem der vorherigen Ansprüche als Mittel zur Nahrungsergänzung.

## Claims

1. Micro-nutrient combination product **characterized in that** the micro-nutrient combination product comprises, based on 100 g of the micro-nutrient combination product, the active agents:
a. 0.6 - 1.2 g lutein;
b. 0.05 - 0.1 g zeaxanthin;
c. 5.6 - 11.2 g vitamin C;
d. 0.8 - 2 g vitamin E;
e. 2.0 - 3.0 mg selen;
f. 0.5 - 1 g zinc.

2. Micro-nutrient combination product of claim 1 **characterized in that** the micro-nutrient combination product is present in solid, liquid and/or gel form, and wherein preferably the micro-nutrient combination product is present in preparation forms selected from the group comprising tablets, capsules, powder, granulate, solutions and/or effervescent tablets.

3. Micro-nutrient combination product of claim 1 or 2 **characterized in that** the micro-nutrient combination product comprises additives promoting bioavailability, solubility and/or dissolution rate.

4. Micro-nutrient combination product of anyone of the preceding claims **characterized in that** the micro-nutrient combination product comprises disintegrants, agents promoting stability, taste masking agents, taste improving agents, fillers, coating agents and/or mould release agents.

5. Micro-nutrient combination product of anyone of the preceding claims **characterized in that** the micro-nutrient combination product comprises, based on 100 g of the micro-nutrient combination product, the active agents:
a. 0.6 g lutein;
b. 0.05 g zeaxanthin;
c. 5.6 g vitamin C;
d. 0.8 g vitamin E;
e. 2.0 mg selen;
f. 0.5 g zinc.

6. Micro-nutrient combination product of anyone of the preceding claims **characterized in that** the micro-nutrient combination product comprises, based on a single dose, the active agents:
a. 3.0 mg lutein;
b. 0.25 mg zeaxanthin;
c. 30,0 mg vitamin C;
d. 4,4 mg vitamin E;
e. 10 µg selen;
f. 2.5 mg zinc.

7. Use of the micro-nutrient combination product of anyone of the preceding claims for the preparation of an agent for dietary prevention and treatment of eye diseases, preferably age-related macular degeneration.

8. Use of the micro-nutrient combination product of anyone of the preceding claims as an agent for dietary supplement.

## Revendications

1. Produit combiné de micronutriment, **caractérisé en ce que** le produit combiné de micronutriment, rapporté à 100 g du produit combiné de micronutriment, comprend les principes actifs :
a. 0,6 à 1,2 g de lutéine
b. 0,05 à 0,1 g de zéaxanthine
c. 5,6 à 11,2 g de vitamine C
d. 0,8 à 2 g de vitamine E
e. 2,0 à 3,0 mg de sélénium
f. 0,5 à 1 g de zinc

2. Produit combiné de micronutriments selon la revendication 1, **caractérisé en ce que** le produit combiné de micronutriments se présente sous forme solide, liquide et/ou de gel, et **en ce que** le produit combiné de micronutriments se présente sous des formes de préparation choisies parmi le groupe comprenant les comprimés, les capsules, de la poudre, du granulé, des solutions et/ou les comprimés effervescents.

3. Produit combiné de micronutriments selon l'une quelconque des revendications précédentes, **caractérisé en ce que** le produit combiné de micronutriments comporte des additifs qui améliorent la biodisponibilité, la solubilité et/ou la vitesse de dissolution.

4. Produit combiné de micronutriments selon l'une quelconque des revendications précédentes, **caractérisé en ce que** le produit combiné de micronutriments comporte des agents de désagrégation, des substances qui améliorent la conservation, des substances masquant le goût, des substances améliorant le goût, des charges, des agents de revêtement et/ou des agents de démoulage.

5. Produit combiné de micronutriments selon l'une quelconque des revendications précédentes, **caractérisé en ce que** le produit combiné de micronutriments comprend, rapporté à 100 g du produit combiné de micronutriments, les principes actifs suivants :
a. 0,6 g de lutéine
b. 0,05 g de zéaxanthine
c. 5,6 g de vitamine C
d. 0,8 g de vitamine E
e. 2,0 mg de sélénium
f. 0,5 g de zinc

6. Produit combiné de micronutriments selon l'une quelconque des revendications précédentes, **caractérisé en ce que** le produit combiné de micronutriments comprend, rapporté à une dose, les principes actifs suivants :
a. 3,0 mg de lutéine
b. 0,25 mg de zéaxanthine
c. 30,0 mg de vitamine C
d. 4,4 mg de vitamine E
e. 10 µg de sélénium
f. 2,5 mg de zinc

7. Utilisation d'un produit combiné de micronutriments selon l'une quelconque des revendications précédentes à la préparation d'un produit pour la prévention diététique et le traitement de maladies oculaires, de préférence en cas de dégénération maculaire liée à l'âge.

8. Utilisation d'un produit combiné de micronutriments selon l'une quelconque des revendications précédentes pour complémenter l'alimentation.
